# EUROPÄISCHE PATENTANMELDUNG

(11) **EP 3 650 118 A1**
(43) Veröffentlichungstag der Anmeldung: **13.05.2020**
(21) Anmeldenummer: 19207751.9
(22) Anmeldetag: 07.11.2019
(51) Int. Cl.: B01J 19/24, B01J 8/02

(54) **KATALYTISCHER MEMBRANREAKTOR ZUR DURCHFÜHRUNG CHEMISCHER GLEICHGEWICHTSREAKTIONEN**

(30) Priorität: 09.11.2018 DE 202018106371 U
(71) Anmelder: MUW SCREENTEC Filter- und Präzisionstechnik aus Metall GmbH, 29378 Wittingen (DE)
(72) Erfinder: GRÜTZNER, Jörg, 16552 Schildow (DE); MARTIN, Dirk, 99085 Erfurt (DE)
(74) Vertreter: Oehmke, Volker

(57) **Zusammenfassung**

Die Erfindung betrifft einen katalytischen Membranreaktor zur Durchführung chemischer Gleichgewichtsreaktionen, insbesondere der Methan- und Methanolsynthese aus Wasserstoff und Kohlendioxid.

Der Erfindung liegt die Aufgabe zugrunde, eine Vorrichtung zur Verfahrensführung von Gleichgewichtsreaktionen vorzuschlagen, bei denen auf der Seite der Reaktionsprodukte Wasser abgetrennt wird und eine verbesserte Ausbeute an Reaktionsprodukten erzielt wird.

Die Aufgabe wird durch einen katalytischen Membranreaktor zur Durchführung chemischer Gleichgewichtsreaktionen, insbesondere der Methan- oder Methanolsynthese, dadurch gelöst, dass in einem Gehäuseoberteil (1) mit einem Reaktionsraum (3), der durch einen lösbaren Gehäusedeckel verschlossen ist, ein Membranrohr (4), welches den Reaktionsraum (3) in eine äußere Kammer (5) und eine innere Kammer (6) unterteilt, angeordnet ist. Um das Membranrohr (4) ist beabstandet ein Korbgeflecht (7) angeordnet, wobei in dem Zwischenraum zwischen Membranrohr (4) und Korbgeflecht (7) eine Katalysatorschüttung (8) vorhanden ist. Für das notwendige Temperaturmanagement sind drei den Reaktionsraum umschließende Ringkammern (11.1, 11.2, 11.3) mit je einem Eingang (12.1, 12.2, 12.3) und Ausgang (13.1, 13.2, 13.3) zum Heizen oder Kühlen des Reaktionsraumes (3) vorhanden.

## Beschreibung

Die Erfindung betrifft einen katalytischen Membranreaktor zur Durchführung chemischer Gleichgewichtsreaktionen, insbesondere der Methan- und Methanolsynthese aus Wasserstoff und Kohlendioxid.

Die Umsetzung von Wasserstoff mit Kohlendioxid wird im Wesentlichen aus zwei Gründen verfolgt. Einerseits wird zunehmend Wasserstoff durch Elektrolyse hergestellt, um Netzschwankungen aus dem zunehmenden Anteil fluktuierenden Stroms aus regenerativen Quellen zu puffern ("power to gas"). Die Speicherung und die Verteilung von Wasserstoff können zu geringen Anteilen direkt im Erdgasnetz erfolgen. Durch die Umsetzung mit Kohlendioxid zu Methan wären die Speicherung und Verteilung in viel größerem Maßstab möglich. Der zweite Grund betrifft die chemische Bindung und Nutzung von Kohlendioxid und hat damit erhebliche umweit- und klimatechnische Vorteile.

In der dem Fachmann bekannten Sabatier-Reaktion, die eine chemische Gleichgewichtsreaktion ist, werden Kohlendioxid (CO₂) und Wasserstoff (H₂) (Edukte) zu Methan (CH₄) und Wasser (H₂O) (Produkte) umgesetzt. Die Sabatier-Reaktion wird meist bei Drücken von ∼1...3 bara (absoluter Druck; bar absolut), vereinzelt auch bei höheren Drücken von bis zu 15 bara, und in einem Temperaturbereich von rund 200 bis 450 °C durchgeführt. Als geeignete Katalysatoren für diese Reaktion sind beispielsweise Nickel, Ruthenium, Rhodium und Cobalt bekannt, die meist auf einem oxidischen Träger wie SiO₂, TiO₂, MgO, Al₂O₃, La₂O₃ aufgebracht sind, wie dies beispielsweise in Ohya et al. 1997: "Methanation of carbon dioxide by using membrane reactor integrated with water vapor permselective membrane and its analysis", Journal of Membrane Science 131 (1-2), beschrieben ist. Am erfolgreichsten stellt sich wohl momentan die Kombination 0,5 Ma% Ru auf TiO₂ dar. Abhängig vom Katalysator können hohe CO₂-Umsätze und CH₄-Ausbeuten bei Temperaturen von ∼300...350°C, erreicht
werden (John Ralston (http://www.pennenergy.com/index/power/display/0723256773/art icles/pennenergy/ugc/renewable/the-sabatier-reaction.html)).

Die Sabatier-Reaktion ist eine sehr anspruchsvolle Reaktion, da sie stark exotherm ist und nach Zugabe der Edukte, je nach Verdünnungsgrad mit inerten Gasen, zu Temperaturen von über 600 °C führt. Dieses Temperaturniveau zerstört nicht nur manche Katalysatoren - beispielsweise gehen aktive Zentren verloren, indem z. B. Ni-Partikel im nm-Bereich zusammensintern - sondern verschiebt auch das Reaktionsgleichgewicht in Richtung der Edukte. Zu niedrige Temperaturen haben dagegen kinetische Limitierungen der Reaktion und der Menge der gewünschten Produkte zur Folge.

Da es sich bei der Sabatier-Reaktion um eine exotherme Gleichgewichtsreaktion handelt, ist sie mit zunehmender Reaktions- bzw. Prozesstemperatur ausbeutelimitiert. Der Entzug eines Reaktionsproduktes direkt aus der Reaktion im Prozess bewirkt eine Erhöhung der Triebkraft zur Produktbildung. Eine geeignete wasserabtrennende Membran bewirkt genau dies, indem das Reaktionsprodukt Wasser direkt im Prozess abgetrennt wird. So wird eine Ausbeutesteigerung verglichen zum Prozess ohne Membran erreicht. Weiterhin wird durch die Abtrennung des Reaktionswassers eine nachgeordnete Trocknung des Methans zur Erreichung der Einspeisequalität idealerweise überflüssig und dieser Prozessschritt kann eingespart werden.

Der Erfindung liegt die Aufgabe zugrunde, eine Vorrichtung zur Verfahrensführung von Gleichgewichtsreaktionen vorzuschlagen, bei denen auf der Seite der Reaktionsprodukte Wasser abgetrennt wird und eine verbesserte Ausbeute an Reaktionsprodukten erzielt wird.

Die Durchführung gekoppelter chemischer Reaktionen unter selektiver Abtrennung eines Reaktionsproduktes unter drastischen Reaktionsbedingungen (> 200 °C, > 10 bar) bedingt den Einsatz spezieller Lösungen bezüglich der eingesetzten Membranen, deren Eindichtung zur Trennung der Gasräume sowie der Gas- und Temperaturführung.

Die Aufgabe wird durch einen katalytischen Membranreaktor zur Durchführung chemischer Gleichgewichtsreaktionen, insbesondere der Methan- oder Methanolsynthese, dadurch gelöst, dass in einem Gehäuseoberteil mit einem Reaktionsraum, der durch einen lösbaren Gehäusedeckel verschlossen ist, ein Membranrohr, welches den Reaktionsraum in eine äußere Kammer und eine innere Kammer unterteilt, angeordnet ist. Um das Membranrohr ist beabstandet ein Korbgeflecht angeordnet, wobei in dem Zwischenraum zwischen Membranrohr und Korbgeflecht eine Katalysatorschüttung vorhanden ist. Für das notwendige Temperaturmanagement sind drei den Reaktionsraum umschließende Ringkammern mit je einem Eingang und Ausgang zum Heizen oder Kühlen des Reaktionsraumes vorhanden. Die innere Kammer weist zur Abführung von Wasser ein Auslassrohr auf und der Reaktionsraum weist zur Aufnahme von Eduktgasen einen Gaseinlass und zur Abgabe von Produktgasen einen Gasauslass auf. Eine vorteilhafte Ausgestaltung besteht darin, dass der Gaseinlass mit einem Einströmrohr in Verbindung steht, welches parallel zum Membranrohr innerhalb der Katalysatorschüttung angeordnet wird. Das Einströmrohr hat über seine gesamte Länge verteilte Öffnungen, so dass somit für eine günstigere Verteilung der Eduktgase gesorgt wird. Eine noch bessere Verteilung der Eduktgase kann erzielt werden, wenn der Gaseinlass mit mehreren Einströmrohren, z. B. über eine Verteilungseinrichtung, in Verbindung gebracht wird, wobei die Einströmrohre vorzugsweise gleichmäßig um das Membranrohr herum innerhalb der Katalysatorschüttung angeordnet werden. Eine weitere zweckmäßige Ausführung ergibt sich, wenn ein Membranrohr zum Einsatz kommt, welches mit einem Katalysator beschichtet ist. Somit würde der erfindungsgemäße Membranreaktor keines Korbgeflechts und keiner Katalysatorschicht mehr bedürfen.

Die Erfindung soll nachfolgend anhand von Ausführungsbeispielen erläutert werden. In den dazugehörigen Zeichnungen zeigen:
- Fig. 1: eine Prinzipdarstellung eines katalytischen Membranreaktors und
- Fig. 2: einen Schnitt durch den erfindungsgemäßen katalytischen Membranreaktor.

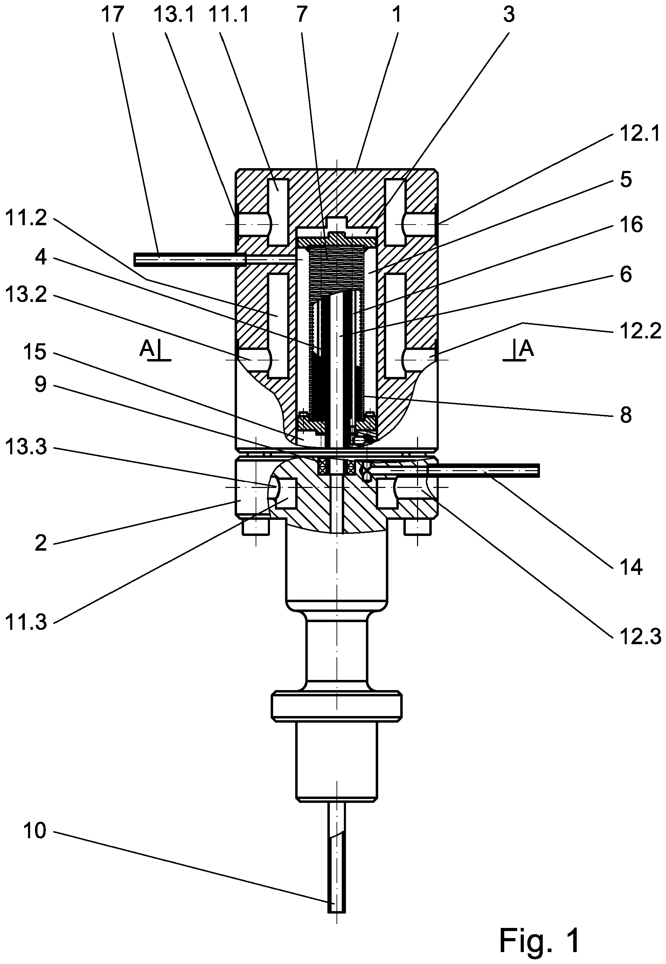

Der katalytische Membranreaktor, bestehend aus einem Gehäuseoberteil 1 und einem Gehäuseunterteil 2 ist in Fig. 1 dargestellt. Im Inneren des Gehäuseoberteils 1 befindet sich der Reaktionsraum 3 und das Membranrohr 4, welches den Reaktionsraum 3 in eine äußere Kammer 5 und eine innere Kammer 6 unterteilt. Ferner ist gemäß Fig. 1 ein Korbgeflecht 7 vorhanden, welches konzentrisch um das Membranrohr 4 angeordnet ist. Der Zwischenraum zwischen Membranrohr 4 und Korbgeflecht 7 ist mit einer Katalysatorschüttung 8 ausgefüllt. Das Gehäuseunterteil 2 ist mit dem Gehäuseoberteil 1 lösbar verbunden, damit das Korbgeflecht 7, die Katalysatorschüttung 8 und das Membranrohr 4 im Reaktionsraum 3 positioniert werden können. Das Membranrohr 4 ist nach Verschließen des Reaktionsraumes 3 mit dem Gehäuseunterteil 2 über eine Eindichtung 9 im Gehäuseunterteil 2 positioniert. Die innere Kammer 6 ist im Bereich der Eindichtung 9 mit einem Auslassrohr 10 verbunden. Für das Temperaturmanagement sind gemäß Fig. 1 drei Ringkammern 11.1, 11.2, 11.3 mit den jeweiligen Eingängen 12.1, 12.2, 12.3 und Ausgängen 13.1, 13.2, 13.3 zu erkennen.

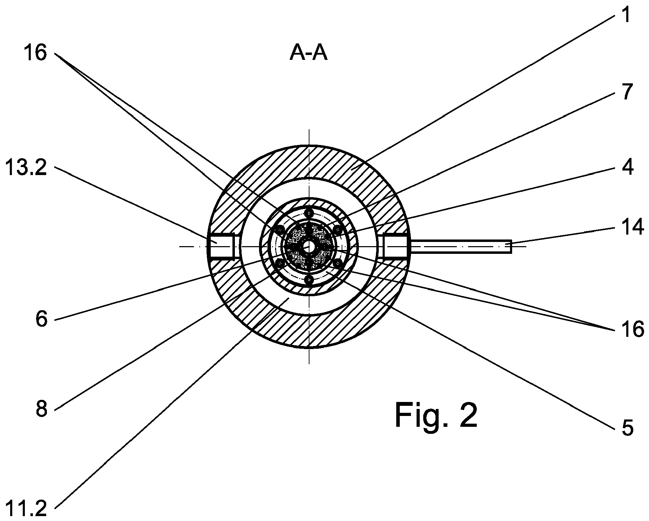

Das im passenden Verhältnis aus Kohlendioxid und Wasserstoff bereitete Eduktgas wird durch den Gaseinlass 14 in die Verteilungseinrichtung 15 geleitet. Von der Verteilungseinrichtung 15 führen vier Einströmrohre 16 ab. Die Einströmrohre 16 sind gemäß Fig. 2 gleichmäßig um das Membranrohr 4 herum und innerhalb der Katalysatorschüttung 8 angeordnet. Die Einströmrohre 16 weisen über ihre gesamte Länge kleine Öffnungen auf, so dass das Eduktgas gleichmäßig verteilt der Katalysatorschüttung 8 zugeführt wird. Sodann erfolgt die Umsetzung des Eduktgases zum Produktgas, welches den Reaktionsraum 3 bzw. die äußere Kammer 5 durch den Gasauslass 17 verlässt. Das mittels der Eindichtung 9 implementierte Membranrohr 4 trennt selektiv eine signifikante Menge Wasserdampf als Teil des entstehenden Produktgases ab, welcher durch das Auslassrohr 10 den Reaktionsraum 3 bzw. die innere Kammer 6 verlässt. Hierdurch wird das chemische Gleichgewicht der Reaktionen beeinflusst und die Katalysatorschüttung 8 zu höheren Umsätzen des Eduktgases bzw. zur Bildung größerer Mengen des Produktgases befähigt. Mittels Fluiden, die über die Eingänge 12.1, 12.2, 12.3 der Ringkammern 11.1, 11.2, 11.3 zugeführt werden und über die Ausgänge 13.1, 13.2, 13.3 den Membranreaktor wieder verlassen, kann ein Heizen und Kühlen des Reaktionsraumes 3 ermöglicht werden. Besonders vorteilhaft erweist es sich, wenn die dritte Ringkammer 11.3, die gemäß Fig. 1 im Gehäuseunterteil 2 angeordnet ist, möglichst nah an der Eindichtung 9 angeordnet wird.

### Bezugszeichenliste

- 1: Gehäuseoberteil
- 2: Gehäuseunterteil
- 3: Reaktionsraum
- 4: Membranrohr
- 5: äußere Kammer
- 6: innere Kammer
- 7: Korbgeflecht
- 8: Katalysatorschüttung
- 9: Eindichtung
- 10: Auslassrohr
- 11.1: Ringkammer
- 11.2: Ringkammer
- 11.3: Ringkammer
- 12.1: Eingang
- 12.2: Eingang
- 12.3: Eingang
- 13.1: Ausgang
- 13.2: Ausgang
- 13.3: Ausgang
- 14: Gaseinlass
- 15: Verteilungseinrichtung
- 16: Einströmrohr
- 17: Gasauslass

## Patentansprüche

1. Katalytischer Membranreaktor zur Durchführung chemischer Gleichgewichtsreaktionen, insbesondere der Methan- oder Methanolsynthese, bestehend aus:
- einem Gehäuseoberteil (1) mit einem Reaktionsraum (3), der durch ein vom Gehäuseoberteil (1) lösbares Gehäuseunterteil (2) verschlossen ist, wobei
- der Reaktionsraum (3) zur Aufnahme von Eduktgasen einen Gaseinlass (14) und zur Abgabe von Produktgasen einen Gasauslass (17) aufweist,
- einem Membranrohr (4), welches den Reaktionsraum (3) in eine äußere Kammer (5) und eine innere Kammer (6) unterteilt, wobei
- die innere Kammer (6) zur Abführung von Wasser ein Auslassrohr (10) aufweist,
- einem konzentrisch um das Membranrohr (4) angeordneten Korbgeflecht (7),
- einer zwischen Membranrohr (4) und Korbgeflecht (7) angeordneten Katalysatorschüttung (8) und
- drei den Reaktionsraum (3) umschließenden Ringkammern (11.1, 11.2, 11.3) mit je einem Eingang (12.1, 12.2, 12.3) und Ausgang (13.1, 13.2, 13.3) zum Heizen oder Kühlen des Reaktionsraumes (3).

2. Katalytischer Membranreaktor nach Anspruch 1, **dadurch gekennzeichnet, dass** der Gaseinlass (14) im Gehäuseunterteil (2) angeordnet ist und im Reaktionsraum (3) endet.

3. Katalytischer Membranreaktor nach Anspruch 2, **dadurch gekennzeichnet, dass** dem Gaseinlass (14) ein Einströmrohr (16) nachgeordnet ist, welches parallel zum Membranrohr (4) zwischen diesem und dem Korbgeflecht (7) angeordnet ist und über seine gesamte Länge Öffnungen aufweist, so dass das Eduktgas besser verteilt an die Katalysatorschüttung (8) heran führbar ist.

4. Katalytischer Membranreaktor nach Anspruch 3, **dadurch gekennzeichnet, dass** zwischen dem Gaseinlass (14) und dem Einströmrohr (16) eine Verteilungseinrichtung (15) mit einem Eingang, der mit dem Gaseinlass (14) verbunden ist, und mehreren Ausgängen angeordnet ist, wobei jedem Ausgang ein Einströmrohr (16) nachgeordnet ist und die Einströmrohre (16) gleichmäßig um das Membranrohr (4) innerhalb der Katalysatorschüttung (8) angeordnet sind.

5. Katalytischer Membranreaktor nach einem der vorhergehenden Ansprüche, **dadurch gekennzeichnet, dass** das Membranrohr (4) über eine Eindichtung (9) mit dem Gehäuseunterteil (2) verbunden ist.

6. Katalytischer Membranreaktor nach Anspruch 5, **dadurch gekennzeichnet, dass** eine der drei Ringkammern (11.3) im Gehäuseunterteil (2) angeordnet ist.

7. Katalytischer Membranreaktor nach Anspruch 6, **dadurch gekennzeichnet, dass** die Ringkammer (11.3) in unmittelbarer Nähe zur Eindichtung (9) angeordnet ist.
